Europäisches Patentamt

European Patent Office

Office européen des brevets

(11) Publication number: **0 216 485**
A1

## (12) EUROPEAN PATENT APPLICATION

(21) Application number: 86306237.8

(22) Date of filing: 12.08.86

(51) Int. Cl.⁴: **C 07 G 15/00**
**A 61 K 37/36, A 61 K 9/00**

(30) Priority: 12.08.85 US 764400

(43) Date of publication of application:
01.04.87 Bulletin 87/14

(84) Designated Contracting States:
AT BE CH DE FR GB IT LI LU NL SE

(71) Applicant: International Minerals And Chemical
Corporation
P.O. Box 207 1401 South Third Street
Terre Haute Indiana 47808(US)

(72) Inventor: Martin, Jerome L.
1334 South Center Street
Terre Haute Indiana 47807(US)

(72) Inventor: Kraemer, John F.
Rural Route No. 21, Box 147
Terre Haute Indiana 47802(US)

(74) Representative: Holmes, Michael John et al,
Frank B. Dehn & Co. European Patent Attorneys Imperial
House 15-19 Kingsway
London, WC2B 6UZ,(GB)

(54) Prolonged release of growth promoting hormones.

(57) The present invention relates to transition metal-complexed growth promoting hormones and to growth promoting formulations containing such a transition metal complex. The invention further relates to a method for promoting the growth of an animal which comprises administering an effective amount of a formulation containing a transition metal-complexed growth promoting hormone.

EP 0 216 485 A1

Croydon Printing Company Ltd

# PROLONGED RELEASE OF GROWTH PROMOTING HORMONES

A major problem associated with the administration of growth hormones to animals is the frequency of treatment required and the resulting rise in labor costs necessitated thereby. Moreover, many animals utilized for human food consumption, particularly ruminants, such as cattle and sheep, are range-fed for extended periods of time prior to feedlot development, thus rendering administration by injection of the hormones virtually impossible, and economically impractical.

One known controlled-release formulation is disclosed in U.S. Patent 4,333,919. This formulation comprises a growth promoting agent admixed with a copolymer derived from lactic acid and glycolic acid and which is substantially free of polymerization catalyst. Examples of the growth promoting agents which are disclosed in this reference include polyethers such as monensin, salinomycin, lasalocid and related compounds. These growth promoters operate by altering the breakdown of food in the rumen of the animal. To be effective, these agents must be deposited in the rumen or recticulo-rumen of the animal. While these growth promoting agents have proved to be effective in promoting growth, it is sometimes desirable to administer to the ruminants physiologically active peptide compositions such as hormones which further promote the growth of the animal.

Unfortunately, these growth promoting hormones are not known to be subject to the controlled release formulations or delivery systems which are introduced into the intestinal tract of animals. Therefore, there exists a need for growth promoting formulations capable of prolonging the release of a growth promoting hormones to ruminants and other animals.

## SUMMARY OF THE INVENTION

The present invention relates to growth promoting formulations capable of prolonging the release of a growth hormone to an animal. More specifically, the formulation concerns a growth promoting formulation comprising an oil vehicle admixed with a transition metal of a growth promoting hormone. The metal complexes of the growth hormone have a lower solubility than the free form of the growth hormone and allow a slower release rate upon injection into the animal.

## DETAILED DESCRIPTION OF THE PREFERRED EMBODIMENT

This invention provides a growth promoting formulation comprising an oil vehicle and an effective amount of a metal complex of a growth promoting hormone. This formulation allows for the delivery of an efficacious dose of a growth promoting agent to the animal over prolonged periods of time following administration of the formulation. The formulation of the present invention contains a transition metal complex of various growth promoting hormones. Suitable growth promoting hormones include physiologically active polypeptide compositions such as human, bovine, equine, ovine, avian and porcine growth promoting hormones.

The growth hormones for use in the present invention can be derived by extraction and subsequent concentration techniques from the pituitary glands of various animals. Growth hormones which are produced by recombinant DNA methods are also suitable. The amino acid sequences of various hormones which are suitable in the present invention are known. For example, the amino acid sequence of human growth promoting hormones is described in an article by C.H. Li in Kirk-Othmer "Encyclopedia of Chemical Technology", 3rd E., Vol. 12, pp. 549-552. The amino acid sequence of bovine growth hormone is described in an article by R.P. Woychik, Nucleic Acid Res., 10, 7197 (1982). The amino acid sequence of ovine growth hormone is described in an article by C.H. Liu et al., Arch. Biochem. Biophys., 156, 493-508 (1973). The amino acid sequence of porcine growth hormone is described in an article by P.H. Seeburg et al., DNA, 2, 37, 45 (1983). All of the above references describing the amino acid sequences are hereby incorporated by reference in their entirety. In addition to the above, one can also form transition metal complexes of growth hormones that have been modified by excission of up to 12 amino acid residues from the amino ends of the amino acid sequences.

Various transition metals can be complexed with the growth-promoting hormones. Examples of such metals include zinc, copper, manganese, cobalt, iron and the like with zinc being preferred. These metal complexes are generally formed by mixing in an aqueous solution the growth promoting hormone with a water-soluble form of the metal; the growth promoting hormone will generally be in undenatured form. Examples of suitable water-soluble forms of transition metals include the acetates, sulfates, bromides, formates, lactates, propionates, chlorides and the like of such metals. Depending on the particular growth promoting hormone, generally speaking, for every mole of growth hormone there should be at least one mole and preferably two or more moles of the transition metal available for complexing with the hormone.

The metal complex of the growth promoting hormone is dispersed in a thickened oil vehicle. As one skilled in the art will appreciate, the oil vehicle must be biocompatible. The oil vehicle serves to provide a carrier for delivery of the metal hormone complex and restrict the complex from any available body fluids upon administration in the animal. Examples of such oils that may serve as vehicles for the present invention include mineral oil and various vegetable oils including, but not limited to, peanut oil, sesame seed oil and soybean oil. Since the metal complexes of the growth hormones are generally solid, there should be sufficient oil to allow the formulation to be injected or introduced to the animal by such means as an implant. Generally, the amount of oil is from about 90 to about 99 weight percent of the thickened oil vehicles. Preferably, the oil comprises from about 95 to about 98 weight percent of the overall formulation.

In addition to the oil, various known adjuvants or excipients may be present to further extend the release rate of the metal-complexed growth hormone. Examples of such adjuvants include beeswax, aluminum monostearate, carnauba, paraffin and the like. These additives are generally present in amounts of from about 1 to about 10 weight percent of the overall formulation. Preferably, the above adjuvants are present in amounts of from about 2 to about 5 weight percent of the overall formulation. Particularly preferred thickened oil vehicles include mixtures of peanut oil and aluminum monostearate and mixtures of soybean oil and beeswax.

A particularly preferred formulation provided by this invention comprises a biodegradable dosage form useful for effecting growth promotion in ruminants and other animals comprising from about 50 to about 0.01 percent by weight, most preferably about 30 to about 0.10 percent of a zinc-complexed growth hormone intimately dispersed throughout a thickened oil vehicle comprising about 90 to about 99 percent by weight of peanut oil and about 1 to about 10 percent by weight of aluminum monostearate.

This invention additionally provides a method for continuously effecting growth promotion in animals comprising administering to a animal an effective amount of a prolonged release formulation made up from about 0.10 to about 30 percent by weight of a transition metal-complexed growth promoting hormone admixed with about 70 to about 99.9 percent by weight of a thickened oil vehicle comprising a mixture of from about 90 to about 99 weight percent of a vegetable oil and from about 1 to about 10 percent of beeswax or aluminum monostearate. According to the method of this invention, animals can receive a continuously effective dose of growth promoting hormone over a prolonged period of time following administration of the formulation. The loading levels of growth hormone are generally suitable for delivering, for example, about 2mg/day for 20 days. A preferred method according to the present invention comprises administering an effective amount of a formulation comprised from about 10 to about 0.5 percent by weight of a metal-complexed growth promoting hormone admixed with about 90 to about 99.5 percent by weight of a thickened oil vehicle comprising a mixture from about 95 to about 98 percent by weight of a vegetable oil and about 2 to about 5 percent of beeswax or aluminum monostearate.

The growth promoting formulations can be administered by a number of methods. One method is by subcutaneous injection. Another method of administration is via an implantation device. There are a number of known implantation devices which can utilize the formulation of the present invention. Implant devices generally contain a pump or matrix whereby the contents of a reservoir are introduced to the surrounding tissues. When the method of administration is via an implanted delivery device, one may elect to eliminate the presence of the oil and simply use the transition metal complex. Optionally, other solid agents, such as the excipients or adjuvants as previously described may be used. In such cases, the transition metal complex may range from about 50 to 99.9 weight percent of the solid growth promoting formulation, with a range of from about 50 to about 80 being preferred.

The following examples are provided to illustrate the present invention and are not, in any way, to be construed as limiting.

EXAMPLE 1

Production of Porcine Growth Hormone-Zinc Complex

Into a 500 ml Erlenmeyer flask were placed 500 mg of porcine growth hormone (Parlow 4640-C) and 400 ml of 0.075 N potassium chloride solution, which had been adjusted to a pH of 7. The mixture was stirred at room temperature for 24 hours (magnetic stirring) and then centrifuged 20 minutes at 9000 rpm to separate a small amount of insoluble material. The supernate was decanted from the insolubles ( 60 mg on drying) and passed through a 0.2 membrane filter to sterilize it.

The porcine growth hormone solution was placed in a 500 ml serum bottle and 20 ml of sterile 0.01 M zinc acetate was added dropwise with magnetic stirring. The mixture was stirred for 15 minutes and the pH was then adjusted to 7.4 with 0.1 N sodium hydroxide. The precipitate and solution were transfered equally to two 250 ml centrifuge bottles and placed in the refrigerator overnight at 5°C. The contents were then centrifuged 20 minutes at 9000 rpm and the supernate was then decanted. The supernate was divided equally between two 500 ml sterile serum bottles, shell frozen, and freeze-dried two days to yield 2.34 g of product. The precipitated zinc-complexed growth hormone (ZnpGH) was slurried in 100 ml sterile de-ionized water, centrifuged for 20 minutes at 9000 rpm and separated from the supernate. The supernate was freeze-dried to yield 36.5 mg of material. The washed ZnpGH was dried under vaccum at room temperature to give 0.290 g of product. The ZnpGH contained 2.35 moles of Zn per mole of pGH and was found to be as active as non-complexed porcine growth hormone in a hypox rate growth assay. The 2.34 g of salts were dissolved in 10 ml water and centrifuged. The supernate was decanted and the precipitate was vacuum-dried to get 240 mg of solid. The various isolated solids were analyzed for protein by the BioRad test and zinc content by AA (atomic absorbtion spectroscopy). The results are given in Table 1.

TABLE 1

Composition of Isolated Solids-ZnpGH Preparation

| Description Fraction | Wt Protein (Via BioRad) | %Zn | Mean Wt Gain Rate Growth Assay |
|---|---|---|---|
| 290 mg ZnpGH | 305.95±36.6 | 0.6998 | 21.2 |
| 36.5 mg from ZnpGH Wash | 9.22± 3.87 | 2.84 | 22.7 |
| 240 mg from conc. supernate | 40.56±27 | 0.8674 | 11.9 (Not Active) |

EXAMPLE 2

pGH-Copper Complex

In a magnetically stirred 50 ml centrifuge tube were placed 50 mg pGH (Parlow 4640-C) and 40 ml 0.075 M KCl. The mixture was stirred for 24 hours at room temperature, and then centrifuged for 20 minutes at 9000 rpm. The supernate was decanted from the insolubles (13.5 mg on drying) and reacted with 2 ml of 0.01 M cupric acetate. The reaction mixture was stored in the refrigerator overnight at 5°C and the copper complex isolated by centrifugation. The complex was washed with 10 ml of water and vacuum dried at room temperature to yield 27.8 mg of the copper-porcine growth hormone complex (CupGH). The CupGH contained 0.6043 % copper via AA, was as active as uncomplexed pGH in the rate growth assay, and contained 61.7% protein via BioRad.

8

## EXAMPLE 3

ZnpGH (264 mg) was pressed into a 1/2-inch pellet using a sterile Perkin-Elmer potassium bromide pellet press at 5000-6000 lbs. The pellet was ground to a powder in a sterile mortar and pestle and sieved through a sterile 60-mesh screen to yield 202 mg fine white zinc complex.

Into a 125 ml serum bottle were placed 5 g of Mallinckrodt N.F. aluminum monostearate and 100 ml of Planter's Peanut Oil (91.6 g). The mixture was heated with magnetic stirring on a hot plate for two hours at 150°C. After cooling to room temperature, the gelled peanut oil was stirred with a spatula and 25 ml (22.34g) of the mixture transferred to a 50 ml beaker. 200mg of the 60-mesh ZnpGH was then added to the peanut oil mixture and stirred at 750 rpm using a 3-blade ss-stirrer until a uniform suspension was obtained (15 minutes). Portions (5 ml) of the suspension were drawn into four sterile 5 ml disposable syringes. The syringes were stored in a freezer unit until the following day.

## EXAMPLE 4

### Animal Study

In order to demonstrate the extended release of the formulations of the present invention, four barrows having a weight of 120 lbs. were injected with 5 ml of a formulation prepared by the procedure in Example 3. Each formulation contained 40 mg of zinc-complexed porcine growth hormone. The 5 ml formulation was injected subcutaneously into the neck of each swine. Blood samples were withdrawn from the jugular vein of the swine and analyzed for growth hormone by radioimmunoassay (RIA). A blood sample was withdrawn before injection to serve as an internal control. Table 2 below lists the results of the radioimmunoassay according to the bleeding dates.

TABLE 2

Blood Concentrations (ng/ml) of Growth Hormone in Swine
After Subcutaneous Administration of a ZnpGH Formulation

| Day | | Pig No. 1 | Pig No. 2 | Pig No. 3 | Pig No. 4 | Mean (pGH) |
|---|---|---|---|---|---|---|
| Prior to Injection | -1 | 3.8 | 1.2 | 2.5 | 5.8 | 7.6 ± 2.15 |
| Prior to Injection | 0 | 1.2 | 4.0 | 3.0 | 7.2 | 8.1 ± 1.7 |
| Post-Injection | 1 | 45.8 | 45.0 | 34.7 | 34.4 | 128 ± 33.1 |
| Post-Injection | 5 | 20.6 | 7.2 | 7.8 | 9.1 | 18.8 ± 7.7 |
| Post-Injection | 7 | 9.4 | 5.2 | 6.3 | 6.6 | 12.5 ± 2.1 |
| Post-Injection | 9 | 7.5 | 5.3 | 8.6 | 10.8 | 13.9 ± 2.0 |

10

## EXAMPLE 5

The following example demonstrates the effectiveness of three different repository systems for the administration of growth hormones (pGH) to swine. Prior to administration of the various formulations, nine barrows were acclimated to confinement and swine diet as listed in the table below.

### Table 3
### Ration Composition

| Item | % |
| --- | --- |
| Ground yellow corn | 73.5 |
| Soybean meal - 44% | 23.5 |
| Dicalcium phosphate | 1.2 |
| Limestone | 0.8 |
| Salt | 0.5 |
| Dawes vitamin premix | 0.5 |
| | 100.0 |
| Calculated composition (as-fed basis) | |
| Crude protein | 16.8 |
| Calcium | 0.62 |
| Phosphorus | 0.54 |

In order to compare the effectiveness of a metal-complexed growth hormone, three different formulations were used. Formulation 1 comprised a mixture of peanut oil and 5% (w/v) of aluminum monostearate containing 8 mg/ml of uncomplexed Parlow swine growth hormone (lot AFP-S324C). Formulation 2 comprised a mixture of soybean oil and 4.5% (w/v) of beeswax containing 8 mg/ml of uncomplexed Parlow swine growth hormone (lot AFP-S324C). Formulation 3 comprised a mixture of peanut oil and 5% (w/v) of aluminum monostearate containing 8 mg/ml of ZnpGH.

Two swine were injected subcutaneously at the base of the ear with 5 ml of compound 1. Three swine were injected with compound 2 in a like manner. Four swine were injected in the same manner with the formulation of compound 3.

Twenty ml of blood were periodically taken from the anterior vena cava of each animal. Serum samples were split and analyzed for serum growth hormone, serum urea nitrogen (BUN).

The results of these blood assays can be seen in Tables 4 and 5 below.

12

TABLE 4

BUN, pGH, and Glucose Concentrations in Swine
Administered Non-Complexed pGH in PAM or Soybean Oil/Beeswax
(Mean Values)

| | Compound 1 pGH/PAM | (SD) | Compound 2 pGH/soybean oil-beeswax | (SD) |
|---|---|---|---|---|
| **Serum Urea Nitrogen (mg/100 ml):** | | | | |
| 0 hours | 11.41 | (0.54) | 13.33 | (0.50) |
| 3 hours | 13.49 | (1.39) | 14.46 | (0.50) |
| 6 hours | 11.79 | (0.24) | 13.21 | (0.48) |
| 24 hours | 4.33 | (0.76) | 5.63 | (0.56) |
| 2 days | 6.54 | (1.18) | 10.18 | (2.03) |
| 3 days | 7.55 | (0.84) | 9.45 | (1.29) |
| 7 days | 11.90 | (0.73) | 12.23 | (0.86) |
| 10 days | 9.69 | (0.37) | 10.16 | (1.34) |
| 15 days | 10.19 | (1.16) | 10.76 | (0.68) |
| **Serum Glucose (mg/100 ml):** | | | | |
| 0 hours | 65.99 | (12.39) | 76.06 | (9.52) |
| 3 hours | 99.08 | (3.03) | 96.55 | (6.89) |
| 6 hours | 118.09 | (13.60) | 105.09 | (6.74) |
| 24 hours | 124.62 | (10.14) | 117.78 | (4.82) |
| 2 days | 93.13 | (14.10) | 94.74 | (12.77) |
| 3 days | 93.13 | (5.59) | 104.61 | (11.09) |
| 7 days | 102.85 | (12.18) | 99.27 | (12.96) |
| 10 days | 77.94 | (7.18) | 92.07 | (4.78) |
| 15 days | 85.91 | (3.32) | 91.73 | (21.27) |
| **Serum Growth Hormone (ng/ml):** | | | | |
| 0 hours | 1.1* | | 3.7 | (2.9) |
| 3 hours | 76.1 | (47.2) | 64.5 | (5.3) |
| 6 hours | 97.5 | (53.2) | 93.0 | (12.8) |
| 24 hours | 84.6 | (37.1) | 57.9 | (4.5) |
| 2 days | 19.5 | (3.5) | 15.7 | (2.8) |
| 3 days | 14.4 | (3.6) | 7.3 | (2.4) |
| 7 days | 1.6 | (0.3) | 0.1* | |

*Value from one sample

13

TABLE 5

BUN, pGH, and Glucose Concentrations in Swine
Administered ZnpGH in PAM
(Mean Values)

| | Compound 3 ZnpGH/PAM | (SD) |
|---|---|---|
| **Serum Urea Nitrogen (mg/100 ml):** | | |
| -1 days | 12.63 | (2.44) |
| 0 hours | 9.30 | (3.35) |
| 24 hours | 7.19 | (1.53) |
| 5 days | 10.49 | (3.41) |
| 7 days | 10.71 | (3.11) |
| 9 days | 16.99 | (4.56) |
| **Serum Glucose (mg/100 ml):** | | |
| -1 days | 102.74 | (4.52) |
| 0 hours | 92.41 | (7.28) |
| 24 hous | 140.30 | (4.64) |
| 5 days | 99.10 | (6.10) |
| 7 days | 94.16 | (11.70) |
| 9 days | 125.22 | (48.93) |
| **Serum Growth Hormone (ng/ml):** | | |
| -1 days | ·3.3 | (2.0) |
| 0 hours | 3.9 | (2.5) |
| 24 hours | 40.0 | (6.3) |
| 5 days | 11.2 | (6.3) |
| 7 days | 6.9 | (1.8) |
| 9 days | 8.1 | (2.3) |

The data of the above tables indicate that the BUN concentrations decrease slightly after injection, returning to the zero hour value after about 7 days, for the three groups of animals. Glucose concentrations were elevated over pre-injection concentrations in most of the blood samples through the final day of bleeding for all three groups. In those pigs which were injected with compounds 1 and 2, serum pGH rose within three hours after injection and remained elevated (over the baseline value) for three days. Serum concentrations of those pigs given the zinc-complexed porcine growth hormone also rose sharply soon after injection and was still found to be elevated in the Day 9 blood samples. Thus, complexing growth hormones with a metal such as zinc in a peanut oil/aluminum monostearate matrix increases the release time of the growth hormone 200% over the release times of non-complexed porcine growth hormones and appears to have no adverse effects on the animals.

JU 50 852

Claims (for the designated states: BE, DE, FR,
        IT, LU, NL, SE, CH and GB)

1.    A transition metal complex of a growth promoting
hormone.

2.    A transition metal complex as claimed in
claim 1 wherein the growth promoting hormone is
selected from porcine, ovine, human, avian, equine
and bovine growth promoting hormones.

3.    A transiton metal complex as claimed in claim 2
wherein the growth promoting hormone is porcine
growth promoting hormone.

4.    A transition metal complex as claimed in
any one of claims 1 to 3 wherein the transition
metal is selected from zinc, copper, manganese,
cobalt and iron.

5.    A transition metal complex as claimed in
claim 4 wherein the transition metal is zinc.

6.    A process for preparing a transition metal
complex as claimed in any one of claims 1 to 5
which comprises mixing in an aqueous solution the
growth-promoting hormone in undenatured form with
a water-soluble form of the metal.

7.    A growth promoting formulation wherein a
transition metal complex as claimed in any one
of claims 1 to 5 is admixed with an adjuvant.

8.    A formulation as claimed in claim 7 wherein
said transition metal complex is present in an
amount of about 50 to about 99.9 weight percent of the overall mixture of adjuvant and transition metal complex.

9.   A formulation as claimed in claim 8, wherein said transition metal complex is present in an amount of about 50 to about 80 weight percent of the overall mixture of adjuvant and transition metal complex.

10.   A growth promoting formulation capable of prolonging the release of a growth promoting hormone comprising an oil vehicle and a transition metal complex as claimed in any one of claims 1 to 5.

11.   A formulation as claimed in claim 10 comprising about 0.10 to about 30 weight percent of a transition metal complex as claimed in any one of claims 1 to 5 and about 70 to about 99.9 weight percent of an oil vehicle which comprises about 90 to about 99 weight percent of an oil selected from mineral oils and vegetable oils and about 1 to about 10 weight percent of an adjuvant.

12.   A formulation as claimed in claim 11 wherein said adjuvant is selected from beeswax, aluminium mononstearate, canauba wax and paraffin.

13.   A formulation as claimed in claim 12 comprising about 0.5 to about 10 percent by weight of a transition metal-complexed growth promoting hormone as claimed in any one of claims 1 to 5 and about 90 to about 99.5 percent by weight of an oil vehicle which comprises about 95 to about 98 percent by weight of a vegetable oil and about 2 to about 5 percent by weight of beeswax or aluminium monostearate.

14.   A formulation as claimed in any one of claims 10 to 13 wherein the oil of said oil vehicle is selected from the group consisting of peanut oil,
sesame seed oil and soybean oil.

15. A formulation as claimed in any one of claims
7 to 14 formed by using a transition metal-complexed
growth promoting hormone prepared by a process
as claimed in claim 6.

16. A method for promoting the growth of an animal
which comprises administering an effective amount
of a transition metal complex as claimed in any
one of claims 1 to 5 or a formulation as claimed
in any one of claims 7 to 15.

Claims (for the designated state AT)

1. A growth promoting formulation containing a transition metal complex of a growth promoting hormone.

2. A formulation as claimed in claim 1 wherein the growth-promoting hormone is selected from porcine, ovine, human, avian, equine and bovine growth promoting hormones.

3. A formulation as claimed in claim 2 wherein the growth-promoting hormone is porcine growth promoting hormone.

4. A formulation as claimed in any one of claims 1 to 3 wherein the transition metal of said transition metal complex is selected from zinc, copper, manganese, cobalt and iron.

5. A formulation as claimed in claim 4 wherein the transition metal of said transition metal complex is zinc.

6. A formulation as claimed in any one of claims 1 to 5 wherein said transition metal complex is admixed with an adjuvant.

7. A formulation as claimed in claim 6 wherein said transition metal complex is present in an amount of about 50 to about 99.9 weight percent of the overall mixture of adjuvant and transition metal complex.

8. A formulaion as claimed in claim 7 wherein said transition metal complex is present in an amount of about 50 to about 80 weight percent of the overall mixture of adjuvant and transition metal complex.

9. A formulation as claimed in any one of claims 1 to 5 comprising said transition metal complex and an oil vehicle.

10. A formulation as claimed in claim 9 comprising about 0.10 to about 30 percent weight percent of said transition metal complex and about 70 to about 99.9 weight percent of an oil vehicle which comprises about 90 to about 99 weight percent of an oil selected from mineral oils and vegetable oils and about 1 to about 10 weight percent of an adjuvant.

11. A formulation as claimed in claim 10 wherein said adjuvant is selected from beeswax, aluminium monostearate, canauba wax and paraffin.

12. A formulation as claimed in claim 11 comprising about 0.5 to about 10 percent by weight of said transition metal complex and about 90 to about 99.5 percent by weight of an oil vehicle which comprises about 95 to about 98 percent by weight of a vegetable oil and about 2 to about 5 percent by weight of beeswax or alumunium monostearate.

13. A formulation as claimed in any one of claims 9 to 12 wherein the oil of said oil vehicle is selected from the group consisting of peanut oil, sesame seed oil and soybean oil.

14. A process for preparing a formulation as claimed in any one of claims 1 to 13 wherein said transition metal complex is formed by mixing in aqueous solution the growth promoting hormone in undenatured form with a water-soluble form of the metal.

15.    A method for promoting the growth of an animal which comprises administering an effective amount of a formulation as claimed in any one of claims 1 to 13.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A- 885 798  (ORGANON) <br><br> * page 1, lines 35-46, 70-78 * <br> --- | 1,2,4-7 | C 07 G  15/00 <br> A 61 K   9/00 <br> A 61 K  37/36 |
| A | US-A-3 803 309  (DESAULLES) <br> * column 2, lines 15-26 * <br> --- | 1,4,5 | |
| A | DE-A-1 467 743  (DUMEX) <br><br> * claims 1, 2 * <br> --- | 1,4,5, 7,10, 12,16 | |
| D,A | US-A-4 333 919  (KLEBER) <br> * column  1, line 13 - column 2, line 18 * <br> ----- | 1 | |

### TECHNICAL FIELDS SEARCHED (Int. Cl.4)

C 07 G  15/00
A 61 K   9/00
A 61 K  37/00

The present search report has been drawn up for all claims

| Place of search | Date of completion of the search | Examiner |
|---|---|---|
| BERLIN | 15-11-1986 | PROBERT C.L. |